# EUROPEAN PATENT APPLICATION

(11) **EP 4 390 383 A1**
(43) Date of publication of application: **26.06.2024**
(21) Application number: 22857746.6
(22) Date of filing: 15.08.2022
(51) Int. Cl.: G01N 27/26

(54) **ELECTROCHEMICAL DETECTION METHOD BASED ON SCREEN-PRINTED ELECTRODE**

(30) Priority: 17.08.2021 CN 202110941071
(71) Applicant: Lansion Biotechnology Co., Ltd., Nanjing, Jiangsu 211100 (CN)
(72) Inventor: XU, Xingshang, Nanjing, Jiangsu 211100 (CN); CHEN, Jeffery, Nanjing, Jiangsu 211100 (CN); WANG, Long, Nanjing, Jiangsu 211100 (CN)
(74) Representative: ZHAOffice SPRL
(86) International application number: PCT/CN2022/112506
(87) International publication number: WO 2023/020434

(57) **Abstract**

The invention relates to an electrochemical detection method based on screen-printed electrodes, which specifically includes the following steps:
Preparation of S1 protein-binding group: The working electrode on the screen-printed electrode substrate is processed, and the protein-binding group is adhered; S2 basic protein reaction: Add basic protein, and the active group on the basic protein reacts with the described The protein-binding group in step S1 reacts; S3 blocking the basic protein: uses a blocking solution to block the basic protein after the reaction in step S2 to obtain a screen-printed electrode to be detected; S4 electrochemical detection: add a detection reagent containing the target protein, the basic protein reacts specifically with the target protein, and the data collected by the working electrode after the reaction are electrochemically analyzed to obtain the detection result. The data is collected for electrochemical analysis, and the results of different detection projects can be obtained based on different basic proteins and target proteins, with high sensitivity.

## Description

### Technical Field

The present invention relates to the technical field of electrochemical detection, and in particular, to an electrochemical detection method based on screen-printed electrodes.

### Background Technique

Electrochemical detection is a method that uses the redox characteristics of substances to determine the characteristic information of the substance to be measured by measuring changes in electrochemical parameters in the solution.

In Chinese patent document CN109490399A, an electrochemical detection device and an electrochemical detection method are disclosed, including: causing the voltage scanning unit to generate voltage scanning signals of multiple different waveforms based on the scanning waveform control signal, and applying the voltage scanning signal to the electrode unit; causing the electrode unit to participate in an electrochemical reaction driven by the voltage scan signal to generate a detection current signal; and causing the detection unit to obtain the detection current signal from the electrode unit. When performing electrochemical detection, an electrical signal with a certain current and potential intensity is generally applied to the solution through an electrode system as an excitation signal to stimulate an oxidation-reduction reaction in the solution, causing corresponding changes in the electrochemical parameters of the solution. By measuring the changes in electrochemical parameters in the solution, the electrochemical detection equipment can accurately determine the characteristic information of the substance to be measured in the solution.

However, in the above technical solution, there is no detailed description of the detection of different items or even multiple items.

### Detailed Description of the Invention

The technical problem to be solved by the present invention is to provide an electrochemical detection method based on screen-printed electrodes, which can carry out different detection projects in complex system samples and has high sensitivity.

In order to solve the above technical problems, the technical solution adopted by the present invention is: the electrochemical detection method based on screen printing electrodes, which specifically includes the following steps:
Preparation of S 1 protein-binding group: The working electrode on the screen-printed electrode substrate is processed, and the protein-binding group is adhered;
S2 basic protein reaction: add basic protein, and the active group on the basic protein reacts with the protein binding group in step S 1;
S3 blocking basic protein: using blocking solution to block the basic protein reacted in step S2 to obtain the screen-printed electrode to be detected;
S4 electrochemical detection: Add a detection reagent containing the target protein, the basic protein reacts specifically with the target protein, and perform electrochemical analysis on the data collected by the working electrode after the reaction to obtain the detection result.

The protein-binding group is adhered and fixed on the surface of the working electrode. When it reacts and combines with the active group on the basic protein, the original membrane charge density will change, eventually leading to a change in membrane potential, which can characterize or define how many base proteins are involved in the reaction and binding with protein binding groups. The function of the blocking solution is to react the unreacted active groups on the basic protein, and then add a permeable protective film to make the binding between active groups on the base protein and protein binding groups more firm. When performing electrochemical detection, the basic protein and the target protein undergo a specific reaction, and the collected data includes the collection of electrodes + electrical signals, especially the impedance or voltage between the working electrode and the reference electrode of the screen-printed electrode. If the difference is poor, electrochemical analysis can be performed, and the results of different detection projects can be obtained based on different basic proteins and target proteins, with high sensitivity.

Preferably, in step S1, the surface of the working electrode is pretreated. The specific method of pretreatment is as follows:
S11 cleans the electrode surface: mechanical grinding and polishing to mirror surface;
S12 Identification of electrode surface cleanliness: Scan in 1×10 -3 mol/L K 3 Fe (CN) 6 phosphate buffer solution until reversible cathode and anode peaks appear, that is, the surface is clean, otherwise return to the steps described S11.

It should be noted that when polishing in step S11, grind in the order of decreasing particle size, and after polishing, move it to an ultrasonic water bath until it is clean.

Preferably, in step S1, the protein-binding group is an aldehyde group, a carboxyl group, or an acyl group.

The aldehyde group can be treated with 25% (molar concentration) glutaraldehyde on the surface of the working electrode substrate as an exposed protein-binding group.

Preferably, the basic protein in step S2 and the target protein in step S4 are corresponding and used for different detection projects.

Preferably, the basic protein in step S2 is a troponin cTnI antibody; the target protein in step S4 is troponin cTnI, which is used for troponin cTnI detection projects.

Troponin is composed of three subunits: troponin I, T, and C. Together with tropomyosin, it regulates the interaction between actin and myosin through the activity of calcium ions on striated actin ATPase. When the myocardium is damaged, the cardiac troponin complex is released into the blood. After 4-6 hours, it begins to rise in the blood. The elevated troponin I can remain in the blood for a long time, 6-10 days. Troponin I has a high myocardial specificity and sensitivity, so troponin I has become the most ideal myocardial infarction marker at present.

Preferably, the basic protein in step S2 is a myoglobin MYO-specific monoclonal antibody; the target protein in step S4 is myoglobin MYO, which is used for myoglobin MYO detection projects.

The MYO method is a two-step enzyme immunoassay based on the "sandwich" principle, in which the sample is incubated with chromium dioxide particles coated with a myoglobin-specific monoclonal antibody to form a particle/MYO complex. The particles are magnetized and separated, and the supernatant is removed. In the second step, the particle/MYO complex is incubated with the conjugation reagent (β-galactosidase-labeled myoglobin-specific monoclonal antibody) to form a particle/MYO/conjugate sandwich. Unbound conjugates and analytes are magnetically separated and removed by washing. Sandwich-bound β-galactosidase and the chromophore substrate chlorophenol red-β-d galactopyranoside (CPRG) catalyze the hydrolysis of the substrate into chlorophenol red (CPR). CPR formation at 577 nm changes in optical absorption in direct proportion to the concentration of MYO present in the patient sample.

Preferably, the basic protein in step S2 is a creatine kinase isoenzyme CK-MB antibody; the target protein in step S4 is a creatine kinase isoenzyme CK-MB, which is used for muscle Acid kinase isoenzyme CK-MB detection projects.

Creatine kinase isoenzyme CK-MB test item: Creatine kinase is a dimeric enzyme, and its subunits include M type (muscle type) and B type (brain type). It is composed of different combinations of three isoenzymes, namely CK-MM, CK-MB, and CK-BB. CK-MM (CK3) is mainly found in striated muscle, with a small amount in myocardium. CK-BB (CK1) mainly exists in brain tissue. The content in other major organs such as liver, kidney, lung, spleen and red blood cells is very small, and it is not easy to detect under normal conditions. CK-MB (CK2) mainly exists in the myocardium and is almost absent in other tissues. In normal serum, there are mainly CK-MM and a small amount of CK-MB, and CK-BB is almost non-existent. A significant increase in CK-MB in the blood indicates myocardial infarction. It is better at judging myocardial damage than total CK activity measurement, and has higher specificity and sensitivity.

There is almost no CK-BB in normal serum. The anti-M subunit antiserum can form an antigen-antibody complex with the M subunit in CK-MM and CK-MB, thereby completely inhibiting the enzyme activity of the M subunit. The measured CK activity value multiplied by 2 represents the activity of CK-MB. The detection principle of creatine kinase (CK) activity is: CK catalyzes the reaction of creatine phosphate and adenosine diphosphate (ADP) to produce adenosine triphosphate (ATP) and creatine; the catalytic reaction of coupling hexokinase (HK) and glucose-6-phosphate dehydrogenase (G6PDH). HK catalyzes the reaction between glucose and ATP to form glucose-6-phosphate, and G6PDH catalyzes the oxidation of glucose-6-phosphate to form 6-phosphoglucolactone and NADPH. The rate of NADPH generation represents CK activity. The reaction principle is as follows:

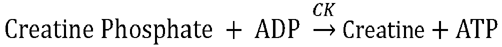

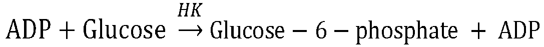

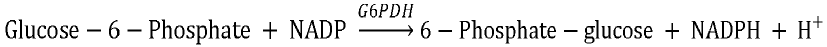

Preferably, the basic protein in step S2 is an N-terminal brain natriuretic peptide precursor NT-proBNP antibody; the target protein in step S4 is an N-terminal brain natriuretic peptide precursor NT-proBNP, which is used for the NT proBNP detection projects of N-terminal brain natriuretic peptide precursor.

ProBNP, BNP, and NT-proBNP coexist in the blood circulation. NT-proBNP is an inactive amino acid fragment, and no clear biological function has been found yet. Left ventricular stretch and ventricular wall tension regulate the release of NT-proBNP. In normal people, NT-proBNP and BNP are always secreted in equal moles, and the plasma concentrations of the two are basically the same. However, in heart failure, the plasma concentration of NT-proBNP is 2-10 times higher than that of BNP, and the half-life is 60 to 120 minutes, which is 20 minutes higher than that of BNP.

The N-terminal brain natriuretic peptide precursor NT-proBNP test item is to detect the concentration of NT-proBNP in human blood. The diagnostic threshold of NT-proBNP value according to age is shown in Table 1 below:

**Table 1 Diagnostic cutoff values of NT-proBNP stratified by age**

| **Age** | <50 years old | 50-75 years old | >75 years old | Judgment criteria |
|---|---|---|---|---|
| **NT-proBNP value** | > 450ng/L | > 900 ng/L | > 1800 ng/L | High likelihood of heart failure |
| | 300-450 ng/L | 300-900ng/L | 300-1800ng/L | Uncertain, diagnose in combination with other clinical manifestations (grey area) |
| | < 300ng/L | < 300ng/L | < 300ng/L | Rule out heart failure |

BNP (type B natriuretic peptide/brain natriuretic peptide) was originally isolated from pig brain tissue and is called brain natriuretic peptide, but its synthesis and secretion are mainly in ventricular myocytes. The BNP gene exists at the end of the short arm of chromosome 1, including 3 exons and 2 introns. It is transcribed through mRNAinto a BNP precursor (proBNP) composed of 108 amino acids, which is present in some endocrine granules of ventricular muscle. When the ventricular volume load or pressure load increases, causing the ventricular muscle to be stretched or the ventricular wall pressure to increase, the stored proBNP is secreted and released, and is quickly decomposed into the inactive amino-terminal proBNP (NT-proBNP) composed of 76 amino acids and BNP composed of 32 amino acids with endocrine activity. The functions of BNP include diuresis, natriuresis, vasodilation and inhibition of the renin-angiotensin-aldosterone system, inhibition of adrenocorticotropic hormone (ACTH) release and sympathetic overreaction, and participation in the regulation of blood pressure, blood volume and salt balance. Recent studies have shown that BNP can also inhibit myocardial fibrosis, vascular smooth muscle cell proliferation and anti-coronary artery spasm.

Preferably, the basic protein in step S2 is serum amyloid A antibody and C-reactive protein antibody; the target protein in step S4 is serum amyloid A and C-reactive protein, which is used for serum amyloid A/C reactive protein detection projects.

SAA: Human serum amyloid A, the precursor of tissue amyloid A, is an acute phase reaction protein. Under normal circumstances, the content in plasma is very small. When the body is stimulated by antigens such as bacteria, viruses, mycoplasma, and chlamydia, liver cells synthesize and secrete a large amount of SAA into the blood, which rapidly increases about 1,000 times within 5-6 hours.

CRP: C-reactive protein, an acute phase response protein synthesized by the liver under stress conditions. Physiological functions include infection defense and phagocytosis and regulation in the inflammatory process. Under normal circumstances, the content in plasma is low. When the body receives infection or trauma, it rises rapidly 5-8 hours and reaches a peak at 24 hours.

During viral infection, SAA changes are more sensitive than CRP changes, so SAA levels can be used as a sensitive and reliable monitoring indicator to judge infection. Joint monitoring of SAA and CRP can make up for the disadvantage that CRP levels do not change significantly during viral infection.

Preferably, the basic protein in step S2 is a procalcitonin PCT antibody; the target protein in step S4 is procalcitonin PCT, which is used for procalcitonin PCT detection projects.

PCT is also called serum calcitonin. It is mainly a peptide hormone extracted from thyroid tumor cell culture fluid. It is a tumor marker in the serum of thyroid tumors. Therefore, the PCT level index of normal people will be low. The detection rate for diagnosis of infectious diseases is relatively high. IL-6 is mainly a kind of interleukin-6, which is a lymphokine of activated T cells and fibroblasts. It can effectively cause the bone marrow cells in the patient's body to gradually grow and differentiate, thereby enhancing the lysis of natural killer cells. Therefore, when patients are infected, their IL-6 indicators will gradually increase. Relevant data have shown that PCT is a calcitonin propeptide substance without hormonal activity. When the PCT value gradually increases, it indicates that the patient has an infectious disease. It is a relatively sensitive indicator for distinguishing infectious diseases.

Procalcitonin (PCT) is a protein synthesized and secreted by the thyroid gland. It can be produced in various tissues under the influence of endotoxin, TNF-α and other cytokines during infection. The level of PCT in plasma increases when severe bacteria, sepsis, and multiple organ failure occur, but does not increase when autoimmunity, allergies, and viral infections occur. PCT reflects the activity of systemic inflammatory response and is the latest specific indicator for early diagnosis, differential diagnosis and treatment monitoring of bacterial infections. Preferably, in step S3, the blocking solution is BSAbovine serum albumin or casein or a protein-free compound.

Preferably, in step S4, the collected data includes the impedance or voltage difference between the working electrode and the reference electrode in the screen-printed electrode.

The technical solution of the present invention is based on the electrochemical detection method of screen-printed electrodes, and detects reagents by detecting electrical signals through electrochemical technology. Reagent reactions are detected by taking place (electro)chemical reactions/interactions and converting them into a detectable analytical signal. Specifically: the sample is fixed on the electrode surface, and then the target molecules are captured on the electrode surface through specific recognition between biomolecules. The basic electrode converts the concentration signal into an electric current, and the measurable electrical signal is used as the corresponding signal, thereby achieving quantitative or qualitative analysis of the target analyte.

### Description of the Drawings

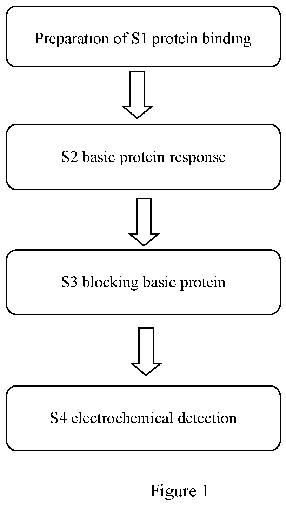

Figure 1 is a flow chart of the electrochemical detection method based on screen-printed electrodes of the present invention.

### Implementation methods

In order to deepen the understanding of the present invention, the present invention will be further described in detail below with reference to the accompanying drawings and examples. The examples are only used to explain the present invention and do not limit the scope of protection of the present invention.

The electrochemical detection method based on screen-printed electrodes in this embodiment, as shown in Figure 1, specifically includes the following steps:
Preparation of S 1 protein-binding group: The working electrode on the screen-printed electrode substrate is processed, and the protein-binding group is adhered;
S2 basic protein reaction: add basic protein, and the active group on the basic protein reacts with the protein binding group in step S 1;
S3 blocking basic protein: using blocking solution to block the basic protein reacted in step S2 to obtain the screen-printed electrode to be detected;
The blocking solution is BSAbovine serum albumin or casein or a protein-free compound;
S4 electrochemical detection: add a detection reagent containing the target protein, the basic protein reacts specifically with the target protein, and perform electrochemical analysis on the data collected by the working electrode after the reaction to obtain the detection result;
The collected data includes the impedance or voltage difference between the working and reference electrodes in the screen-printed electrodes.

The basic protein in step S2 and the target protein in step S4 are corresponding and are used for different detection projects. Different detection projects correspond to different target proteins according to different basic proteins. For example, (1) the basic protein in step S2 is a troponin cTnI antibody; the target protein in step S4 is troponin cTnI, which is used for troponin cTnI detection projects; (2) The basic protein in step S2 is a myoglobin MYO-specific monoclonal antibody; the target protein in step S4 is myoglobin MYO, which is used for myoglobin MYO detection projects; (3 ) The basic protein in step S2 is a creatine kinase isoenzyme CK-MB antibody; the target protein in step S4 is a creatine kinase isoenzyme CK-MB, which is used for creatine kinase Isoenzyme CK-MB detection projects; (4) The basic protein in step S2 is N-terminal brain natriuretic peptide precursor NT-proBNP antibody; the target protein in step S4 is N-terminal brain natriuretic peptide precursor NT-proBNP antibody, which is used for N-terminal brain natriuretic peptide precursor NT-proBNP detection projects; (5) the basic protein in step S2 is serum amyloid A antibody and C-reactive protein antibody; the target protein in step S4 is serum amyloid A and C-reactive protein, which is used for serum amyloid A/C-reactive protein detection projects; (6) the basic protein in step S2 is procalcitonin PCT antibody; the target protein in step S4 is procalcitonin PCT, which is used for procalcitonin PCT detection projects.

In step S1, the surface of the working electrode is pretreated. The specific method of pretreatment is:
S11 cleans the electrode surface: mechanical grinding and polishing to mirror surface;
S12 Identification of electrode surface cleanliness: Scan in 1×10 -3 mol/L K 3 Fe (CN) 6 phosphate buffer solution until reversible cathode and anode peaks appear, that is, the surface is clean, otherwise return to the steps described S11.

In step S1, the protein-binding group is an aldehyde group, a carboxyl group, or an acyl group; the aldehyde group can be treated with 25% (molar concentration) glutaraldehyde on the surface of the working electrode substrate as the exposed protein-binding group.

For those of ordinary skill in the art, the specific embodiments are only illustrative descriptions of the present invention. Obviously, the specific implementation of the present invention is not limited by the above manner, as long as the method concept and technical solution of the present invention are adopted for various operations. Non-substantive improvements, or direct application of the concepts and technical solutions of the present invention to other situations without improvement, such as replacing the basic protein and target protein for other detection projects, are within the scope of the present invention.

## Claims

1. An electrochemical detection method based on screen-printed electrodes, **characterized in that** it specifically includes the following steps:
Preparation of S1 protein-binding group: The working electrode on the screen-printed electrode substrate is processed, and the protein-binding group is adhered;
S2 basic protein reaction: add basic protein, and the active group on the basic protein reacts with the protein binding group in step S1;
S3 blocking basic protein: using blocking solution to block the basic protein reacted in step S2 to obtain the screen-printed electrode to be detected;
S4 electrochemical detection: Add a detection reagent containing the target protein, the basic protein reacts specifically with the target protein, and perform electrochemical analysis on the data collected by the working electrode after the reaction to obtain the detection result.

2. The electrochemical detection method based on screen-printed electrodes according to claim 1, **characterized in that**, in the step S1, the surface of the working electrode is pre-treated, and the specific method of pre-treatment is as follows:
S11 cleans the electrode surface: mechanical grinding and polishing to mirror surface;
S12 Identification of electrode surface cleanliness: Scan in 1×10 -3 mol/L K 3 Fe (CN) 6 phosphate buffer solution until reversible cathode and anode peaks appear, that is, the surface is clean, otherwise return to the steps described S11.

3. The electrochemical detection method based on screen-printed electrodes according to claim 2, **characterized in that** in step S1, the protein-binding group is an aldehyde group, a carboxyl group, or an acyl group.

4. The electrochemical detection method based on screen printing electrodes according to any one of claims 1 to 3, **characterized in that** the basic protein in step S2 and the target protein in step S4 are corresponding and used for different detection projects.

5. The electrochemical detection method based on screen-printed electrodes according to claim 4, **characterized in that** the basic protein in step S2 is a troponin cTnI antibody; the target protein in step S4 is troponin cTnI, which is used in troponin cTnI detection projects.

6. The electrochemical detection method based on screen-printed electrodes according to claim 4, **characterized in that** the basic protein in step S2 is a myoglobin MYO-specific monoclonal antibody; the target protein in step S4 is myoglobin MYO, which is used in myoglobin MYO detection projects.

7. The electrochemical detection method based on screen-printed electrodes according to claim 4, **characterized in that** the basic protein in step S2 is a creatine kinase isoenzyme CK-MB antibody; the target protein in step S4 is creatine kinase isoenzyme CK-MB, which is used in the creatine kinase isoenzyme CK-MB detection projects.

8. The electrochemical detection method based on screen-printed electrodes according to claim 4, **characterized in that** the basic protein in step S2 is an N-terminal brain natriuretic peptide precursor NT-proBNP antibody; the target protein in step S4 is N-terminal brain natriuretic peptide precursor NT-proBNP, which is used in the N-terminal brain natriuretic peptide precursor NT-proBNP detection projects.

9. The electrochemical detection method based on screen-printed electrodes according to claim 4, **characterized in that** the basic protein in step S2 is serum amyloid A antibody and C-reactive protein antibody; the target proteins in S4 are serum amyloid A and C-reactive protein, which are used in serum amyloid A/C-reactive protein detection projects.

10. The electrochemical detection method based on screen-printed electrodes according to claim 4, **characterized in that** the basic protein in step S2 is a procalcitonin PCT antibody; the target protein in step S4 is procalcitonin PCT, which is used in procalcitonin PCT detection projects.

11. The electrochemical detection method based on screen-printed electrodes according to claim 4, **characterized in that** in step S3, the blocking solution is BSA bovine serum albumin or casein or a protein-free compound.

12. The electrochemical detection method based on screen-printed electrodes according to claim 5, **characterized in that** in step S4, the collected data includes the impedance or voltage difference between the working electrode and the reference electrode in the screen-printed electrode.
